# EUROPEAN PATENT SPECIFICATION

(11) **EP 3 180 068 B1**
(45) Date of publication and mention of the grant of the patent: **07.11.2018**
(21) Application number: 16745822.3
(22) Date of filing: 17.06.2016
(51) Int. Cl.: A61M 39/22, A61M 25/00

(54) **INTRAVENOUS CATHETER APPARATUS WITH AN INTEGRATED THREE WAY STOP COCK**
INTRAVENÖSE KATHETERVORRICHTUNG MIT INTEGRIERTEM DREIWEGE-ABSPERRHAHN
APPAREIL À CATHÉTER INTRAVEINEUX AVEC UN ROBINET D'ARRÊT TROIS VOIES INTÉGRÉ

(30) Priority: 01.07.2015 IN 1983DE2015
(43) Date of publication of application: 21.06.2017
(73) Proprietor: Poly Medicure Limited, Faridabad, Haryana 121004 (IN)
(72) Inventor: BAID, Rishi, New Delhi 110048 (IN)
(74) Representative: Thum, Bernhard
(86) International application number: PCT/IB2016/053615
(87) International publication number: WO 2017/001963

(56) References cited:
- EP-A1- 2 168 628
- EP-A2- 0 943 356
- CN-U- 2 116 492
- GB-A- 1 024 410
- GB-A- 1 344 166
- US-A- 3 934 576
- US-A1- 2010 298 782

## Description

### FIELD OF THE INVENTION

The present invention relates to a stopcock for use in intravenous catheter apparatus and infusions of intravenous fluid to a patient. More particularly, the present invention relates to an intravenous catheter apparatus with an integrated stopcock having improved fluid flow and control capabilities.

### BACKGROUND OF THE INVENTION

The use of a stopcock in the medical field, when a blood transfusion or a fluid/solution transfusion is carried out, is known. Stopcocks provide a quick and sterile way for diverting intravenous fluid flow or medication into a patient by changing the flow path in the IV line system. For example, a conventional three-way stopcock typically comprises a cylindrical portion having three branch tubes projecting in a T-like shape at the outer periphery of the cylindrical portion. A plug portion is inserted in and rotatably attached to the cylindrical portion and having a liquid path in a T-like shape corresponding to the branch tubes of the cylindrical portion. A lever or valve is attached to the plug portion for switching the flow path. The lever or valve is integrally formed with the plug portion or fixed to the plug portion which may be used for stopping or regulating the flow of a fluid. The term "fluid" as used herein may include liquids and/or gasses.

Stopcocks have been used with increasing frequency as a needle-less intravenous injection port. Once an initial IV injection port is opened using a needle, subsequent injections and infusions are possible through the same injection port via a stopcock with more than one port, for example, a three-way stopcock having three ports separated by a shut-off valve. As such the stopcocks provide an inexpensive method of avoiding needle-stick injuries and for a clinician to attain use of needle-less injection techniques whenever possible for intravenous fluid administration. Intravenous catheter apparatus are well known in the art and are widely used to deliver intravenous fluids and medications to patients every day. Such intravenous catheter apparatus are available with or without needle guards.

An intravenous catheter apparatus according to the preamble of claim 1 is disclosed in GB 1 344 166 A.

The present invention relates to a three-way stopcock providing improved fluid flow and control capabilities useful in the medical field, which is integrally made in an intravenous catheter apparatus for administration of intravenous fluids.

### SUMMARY AND OBJECTS OF THE INVENTION

An object of the present invention is to provide an improved intravenous catheter apparatus with an integrated three-way stopcock.

Accordingly, the present invention relates to an intravenous catheter apparatus having the features of claim 1.

The intravenous catheter apparatus has an integrated three-way stopcock assembly and a catheter hub assembly having a proximal end configured to be connected to a needle hub having a needle, and having a distal end configured to be connected to a catheter tube, wherein a straight needle is capable of passing through said proximal end and distal end of the catheter hub assembly, in a ready position of the intravenous catheter apparatus, said catheter hub assembly includes: a wing housing at the distal end, a housing at the proximal end, and a body portion in between housing the three-way stopcock assembly; wherein said body portion comprises an entry port in communication with said housing, an exit port in communication with said wing housing, wherein the entry port and the exit port extend along a horizontal line in an axial direction, and an injection port, wherein all of these ports are confluent at a central chamber configured within the body portion; wherein said three-way stopcock assembly has a top portion and an opposing bottom portion, both along a vertical line perpendicular to the horizontal line, as well as a handle configured in said top portion, wherein the handle has a first portion parallel to the horizontal line and a second portion parallel to the injection port, in the ready position, and wherein the three-way stopcock assembly has rotation capabilities relative to the body portion of the catheter hub assembly.

The injection port may extend in a plane with the entry port and the exit port. This would make manufacturing the ports easier.

The injection port may extend at an acute angle to the entry port. Such an arrangement of the injection port away from a patient is more convenient for the practitioner.

Alternatively, the injection port may extend perpendicular to the entry port.

The three-way stopcock assembly may have 360° rotation capabilities with regard to the body portion of the catheter hub assembly.

The three-way stopcock assembly may have only 180° rotation capabilities with regard to the body portion of the catheter hub assembly. For achieving all of the possible flow configurations through the ports, 180° are not only enough, but a particular limitation, by abutment for example, to these 180° provides feedback during the rotation of the three-way stopcock assembly with regard to the body portion of the catheter hub assembly which is useful for the practitioner.

The body portion may be molded as one piece.

The bottom portion of the three-way stopcock assembly may be cylindrical, which is structurally beneficial to the provision of rotation capabilities.

The central chamber may be defined within the bottom portion of the three-way stopcock assembly. This allows relative rotational movement of the three-way stopcock assembly with regard to the catheter hub assembly to open and close flow paths communicating with the central chamber.

A direction of extension of the first portion of the handle may correspond to a direction of extension of a first opening and a second opening in the bottom portion of the three-way stopcock assembly, and a direction of extension of the second portion of the handle may correspond to a direction of extension of a third opening in the bottom portion of the three-way stopcock assembly. This way, without being able to actually see the openings hidden by the catheter hub assembly of the intravenous catheter apparatus, their direction or extension and angular location can be derived from the handle.

In the ready position of the intravenous catheter apparatus, the entry port and the first opening, the exit port and the second opening, as well as the injection port and the third opening may form respective first, second and third flow paths. The intravenous catheter apparatus provides a further orientation of the three-way stopcock assembly which blocks at least the first and second flow paths, and at least one further orientation which allows flow between two of the first, second and third flow paths via the central chamber.

A bore may be provided in the handle along the vertical line, wherein the bore has an opening in a top portion thereof defining a top port and communicating with the central chamber.

The bore may be a blind hole with a side opening at the bottom which communicates with the central chamber.

A flow regulating member may be arranged in between the opening of the top port and the central chamber, such that it allows fluid passage only from the outside to the central chamber and prevents back flow of fluid from the central chamber to the outside.

The flow regulating member may be an elastic element covering the side opening from the central chamber side of the blind hole.

The catheter hub assembly may have at least one protrusion in a bottom portion extending within the bottom portion of the catheter hub assembly and towards the flow regulating member capable of preventing an accidental vertical displacement of the flow regulating member and uncovering of the side opening.

The at least one protrusion may be offset from the horizontal line in order to allow passage of a needle through the central chamber, in the ready position.

The catheter hub assembly may have two protrusions parallel to each other.

The intravenous catheter apparatus may have a click-feature between the three-way stopcock assembly and the catheter hub assembly.

The click-feature may have only as many engaged positions as there are rotationally selectable ports. This way feedback is given only when at least one flow path is open.

The click-feature may have only one more engaged positions as there are rotationally selectable ports. The additional engage position may correspond to a blocking of all three flow paths.

An inner wall of the catheter hub assembly may be provided with at least one projection and grooves matching with said at least one projection may be provided in an outer wall of the three-way stopcock assembly.

The three-way stopcock assembly may be rotatable in uniform steps within the body portion of half the angle between the injection port and the entry port.

An inner wall of the catheter hub assembly may be provided with grooves and at least one projection matching with said grooves may be provided in an outer wall of the three-way stopcock assembly.

The three-way stopcock assembly may be snap-fitted inside the catheter hub assembly.

The snap-fit may be formed by a protrusion ring and a corresponding ring groove.

The protrusion ring may comprise the grooves and the ring groove may comprise the at least one projection.

The number of projections may be equal to the number of grooves.

A bottom face of the handle may be provided with a mating member and grooves matching with said mating member may be arranged in a top face of the body portion.

### BRIEF DESCRIPTION OF THE ACCOMPANYING DRAWINGS:

The foregoing and other objects, features, and advantages of the invention will be apparent from the following detailed description taken in conjunction with the accompanying drawings, wherein:
Figs. 1, 2 and 3 are respectively a perspective view, a cross-sectional side view and an exploded cross-sectional side view of an intravenous catheter apparatus with an integrated three-way stopcock assembly according to the present invention;
Figs. 4A and 4B are cross-sectional side views of a catheter hub assembly with the integrated three-way stopcock assembly showing fluid flow paths according to the present invention;
Fig. 5A, 5B, 5C and 5D are top-views of the intravenous catheter apparatus showing the rotational positions of a handle of the integrated three-way stopcock assembly according to the present invention;
Figs. 6A, 6B and 6C are respectively a perspective view, a top view and a cross-sectional side view of the catheter hub assembly according to the present invention;
Figs. 7A and 7B are respectively a perspective view and a cross-sectional side view of the three-way stopcock assembly according to the present invention;
Fig. 8 is a bottom view of a base portion of a body portion of the catheter hub assembly according to the present invention;
Fig. 9 is a cross-sectional side view of the body portion of the catheter hub assembly with the handle and injection port according to a modification of the present invention.

### DETAILED DESCRIPTION OF THE INVENTION

Embodiments of the presently disclosed invention will now be described in detail with reference to the drawings, wherein like reference numerals designate identical or corresponding elements.

In the drawings and in the description, the term "proximal" refers to a region of the device or parts thereof or a location on the device which is closest to, for example, a user using the device. In contrast to this, the term "distal" refers to a region of the device which is farthest from the user, for example, the distal region of a needle will be the region of a needle containing the needle tip which is to be inserted e.g. into a patient's vein.

The entire device is shown in drawings for illustration only. To achieve the various rotational position of the handle of the device, it is to be understood that the needle hub is not attached with the catheter hub assembly.

The terms "axial portion" used with respect to the three-way stopcock described herein refers to a positional relationship of a direction of the flow path being parallel to the needle or in particular being in a direction of the axis of the needle. A "horizontal portion" is defined as a positional relationship in the direction perpendicular to the "axial portion".

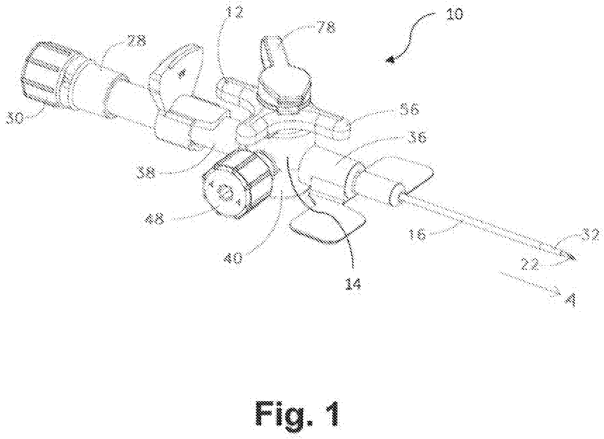

Referring to Figs. 1 to 3, an intravenous catheter apparatus 10 with an integrated three-way stopcock assembly 12 in accordance with the invention is illustrated. The intravenous catheter apparatus 10 includes a catheter hub assembly 14, a catheter tube 16 and a needle 18 connected to a needle hub 28. The needle 18 has a needle shaft 20, a needle tip 22 at a distal section 24 of the needle shaft 20. The needle hub 28 is attached to a proximal end 30 of the needle shaft 20 opposite a distal end 32. An enlargement (not shown) of the needle 18 is provided between the distal section 24 and a proximal section 26 of the needle shaft 20. The enlargement has a maximum dimension in a direction transverse to the needle shaft 20, which is greater than the outer diameter of the distal and proximal sections 24, 26. The enlargement can be made, for example, by crimping the needle shaft 20.

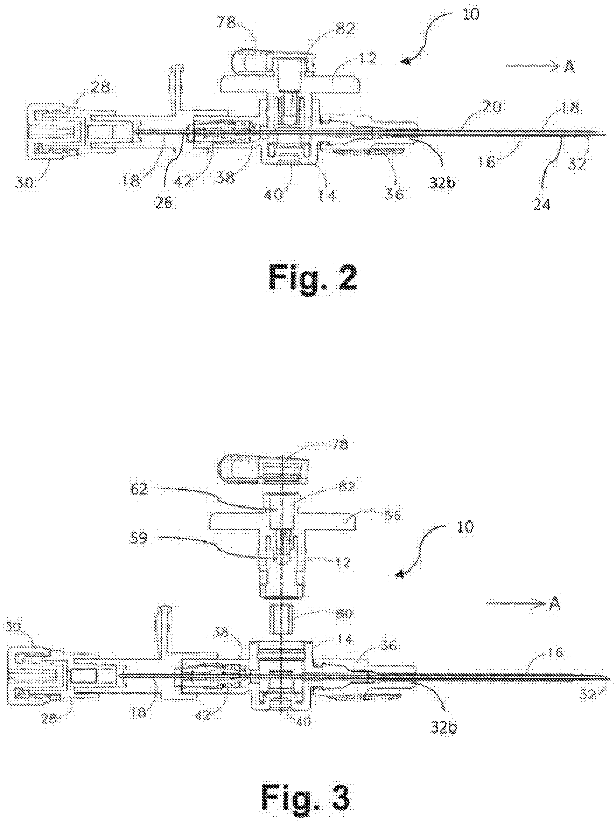

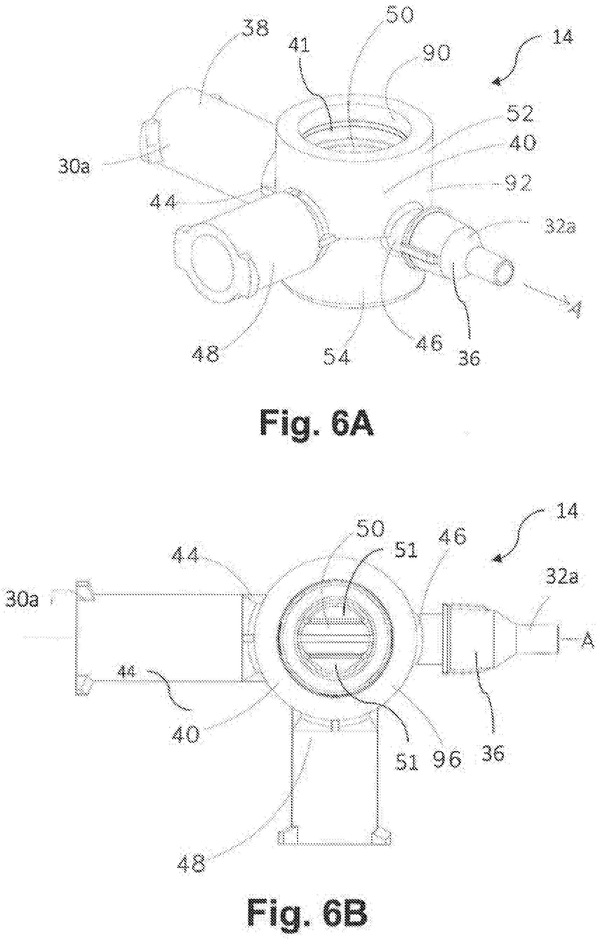

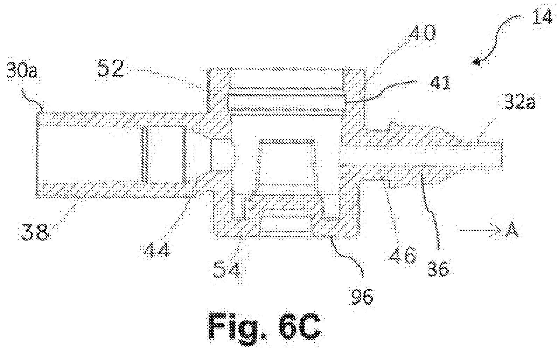

As shown in Figs. 6A, 6B and 6C, the catheter hub assembly 14 has a distal end 32a and a proximal end 30a. The catheter hub assembly 14 includes a wing housing 36 at the distal end 32a, a housing 38 at the proximal end 30a, and a body portion 40 in between configured to house the three-way stopcock assembly 12 in between the housing 38 and the wing housing 36. The catheter tube 16 is arranged adjacent to a distal end 32b of the wing housing 36 (see Figs. 2 and 3). The housing 38 is configured to receive a needle guard 42 (see Figs. 2 and 3) which is movably arranged on the needle shaft 20. The wing housing 36 is illustrated without wings in Figs. 6A to 6C.

The body portion 40 is molded as one piece. The body portion 40 comprises an entry port 44 in communication with the housing 38, an exit port 46 in communication with the wing housing 36, and an injection port 48. The ports 44, 46 and 48 are confluent at a central chamber 50 configured within the body portion 40. The central chamber 50 is configured to receive a three-way stopcock assembly 12. The injection port 48 protrudes from a middle region of central chamber 50 perpendicularly to an axial direction A formed by the entry port 44 and the exit port 46 along a horizontal line. The injection port 48 is used for adding medication or fluids to the IV system. The body portion 40 further has a ring groove 41 along its inner circumference for the purpose of engaging in a snap-fit with the three-way stopcock assembly 12. Such snap-fit may be detachable or permanent once engaged and will we described below in further detail. The catheter hub assembly 14 has two parallel protrusions 51 in a bottom portion 54 extending therefrom towards a top portion 52 of the catheter hub assembly 14. The protrusions 51 are arranged to be distanced from another in order to not obstruct the horizontal line in the axial direction A, in which the entry port 44 and the exit port 46 extend. The purpose of these protrusions 51 is explained further below.

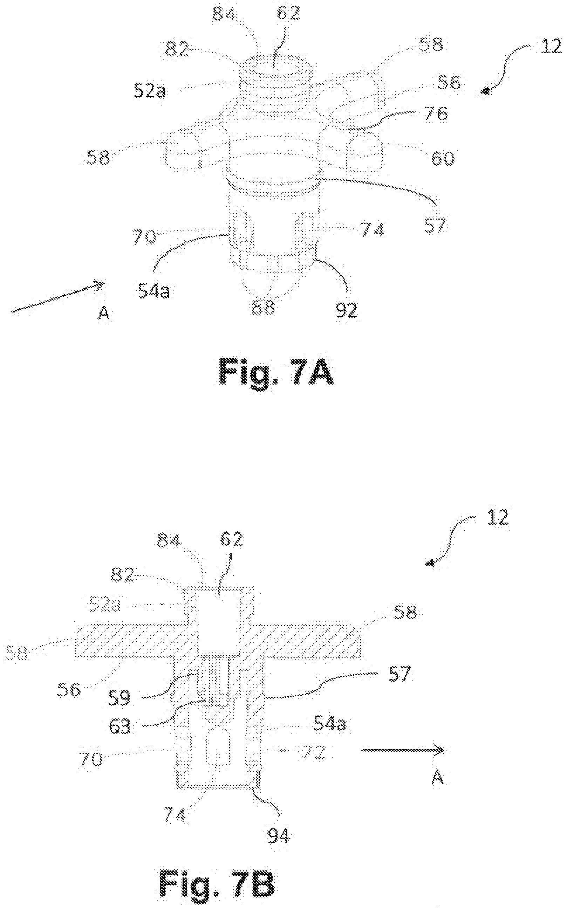

Referring now to Figs. 7A and 7B, the three-way stopcock assembly 12 has a top portion 52a and an opposing bottom portion 54a. A handle 56 is configured in the top portion 52a having an axial portion 58, further referred to as a first portion 58, and a horizontal portion 60, further referred to as a second portion 60, with 360° rotation capabilities. Beneath the handle 56 is a circumferential protrusion ring 57 corresponding to the circumferential ring groove 41 of the catheter hub assembly 14 in order to achieve a snap-fit connection between the three-way stopcock assembly 12 and the catheter hub assembly 14. As will become apparent from the further explanation with regard to the function of the intravenous catheter apparatus 10, having only 180° rotation capabilities are also possible.

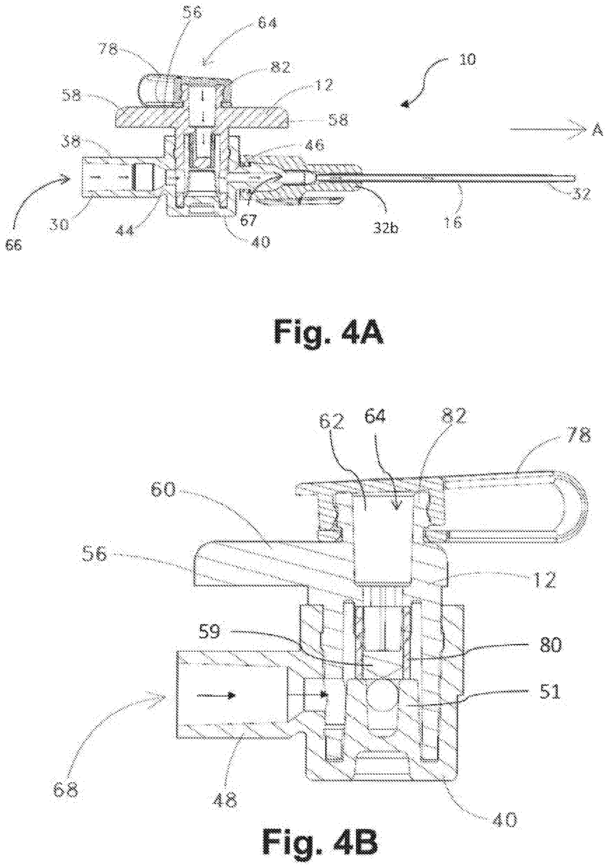

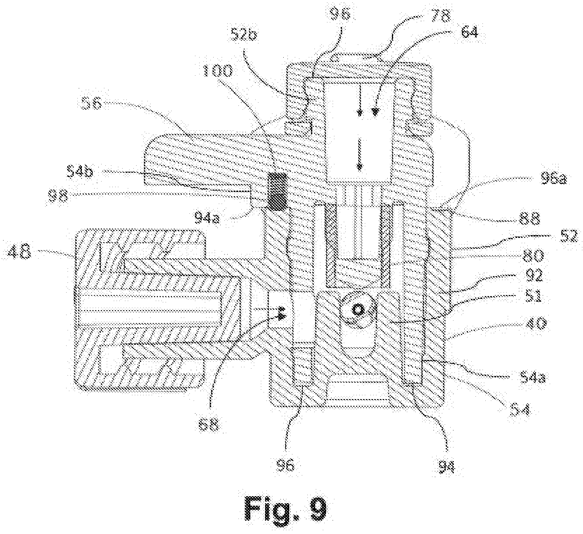

A bore 62 in the handle 56 being in the vertical direction and thus perpendicular to the horizontal line in the axial direction A defines a first flow path 64 which is covered by a cap 78 (see Figs. 4A, 4B and 9, for example). The bore 62 has an opening 84 in a top portion 52b of the handle 56 which is in communication with the central chamber 50 and defines a top port 82. In this embodiment, the bore 62 is a blind hole with a side opening 63 at the bottom, which side opening 63 communicates with the central chamber 50.

The first portion 58 of the handle 56 is associated with a second flow path 66 and a third flow path 67 (see Fig. 4A). A fourth flow path 68 (see Fig. 4B) is associated with the second portion 60 of the handle 56. The second 66 to fourth 68 flow paths, not being limited to, form a "T" configuration. The bore 62 opens in the top portion 52a of the three-way stopcock assembly 12. The second 66, third 67, and fourth 68 flow paths are formed by alignment of the entry port 44 and the first opening 70, the exit port 46 and the second opening 72, and the injection port 48 and the third opening 74, respectively in the bottom portion 54a of the three-way stopcock assembly 12.

The first 64, second 66, third 67 and fourth 68 flow paths, thus, include at least four flow passages having three inlet passages and one outlet passage i.e. through exit port 46.

As shown in Figs. 2 and 3, prior to the use of the intravenous catheter apparatus 10, the needle guard 42 is arranged in the housing 38 of the catheter hub assembly 14. In this situation, the needle 18 extends completely through the needle guard 42, thereby deflecting the first arm of the needle guard 42 outwards, i.e. at an angle to the axial direction A, such that a distal wall of the first arm is supported on the needle shaft 20. Following the insertion of the catheter tube 16 into a patient, the needle 18 is withdrawn from the catheter tube 16 and the needle shaft 20 moves through the needle guard 42 while the needle guard 42 is still retained in the catheter hub assembly 14. Once the needle tip 22 passes the transverse distal wall of the needle guard 42, i.e. such that the needle shaft 20 no longer supports the distal wall, a restoring force ensures that the first arm of the needle guard 42 is moved back into alignment with the axial direction A of the needle guard 42, so that the needle tip 22 is blocked by a distal wall of the needle guard 42, i.e. the needle tip 22 is prevented from axially projecting out of the needle guard 42 and is pulled out safely while being protected by the needle guard 42. In alternative embodiments, the intravenous catheter apparatus 10 with an integrated three-way stopcock assembly 12 can be configured without a needle guard 42.

Removal of the needle 18 from the catheter hub assembly 14 makes the handle 56 rotatable allowing the passage of fluid through first 64, second 66, third 67, and fourth 68 flow paths. The rotation of the handle 56 controls the locking and unlocking of the second 66 to fourth 68 flow paths by rotational displacement of the first 70, second 72 and third 74 openings with regard to the respective entry 44, exit 46, and injection 48 ports.

A flow regulating member 80 as shown in Figs. 3 and 4B, which is preferably made of silicone material is arranged inside the central chamber 50 on a blind hole protrusion 59 of the three-way stopcock assembly 12 as shown in Fig. 7B such that it covers the side opening 63 at the bottom of the blind hole protrusion 59 communicating with the central chamber 50. The flow regulating member 80 allows the fluid to pass through only in one direction i.e. from outside to the inner central chamber 50 and prevents back flow of fluid from the central chamber 50. Referring to Fig. 4B, when the fluid is infused through the top port 82 by a standard syringe, the taper of the syringe fits with the passage of the top port 82 and applied fluid pressure causes the flow regulating member 80 to open by being widened and infuse the fluid into the central chamber 50 and thus into a patient. The passage of the top port 82 is not affected by the rotation of the handle 56 and is therefore never blocked. After infusion of the fluid the flow regulating member 80 is adapted to regain its original position and block the first flow path 64 and thus prevent the backflow of the fluid.

The flow regulating member 80 can be arranged by heat sealing, adhesive sealing, ultrasonic welding, heated die, radio frequency, mechanical seal, insert molding, laser welding, press/snap fit, annular ring with groove fitment etc. The flow regulating member 80 is made of a flexible material and may be a sheet or film, tube or fiber, or plug form. The material of the member 80 may be a fabric, such as a nonwoven, woven, or knit fabric, or a scrim. The member 80 may be made of paper, such as filter paper, or a cloth, or a metal mesh. It can also be made of fiberglass, cellulosic, ceramic or the like. The member 80 can also be a porous polymeric film or membrane, synthetic or natural, where the pores form the interstices or passageways. Representative polymers useful for the material include polyamide, polyurethane, polyester, polycarbonate, polyvinylidene fluoride, polyacrylic, polyolefins, such as polyethylene and polypropylene, polytetrafluoroethylene, polyvinyl chloride and the like.

As shown in Figs. 4A and 4B, the first flow path 64 is in communication with the second 66, third 67 and fourth 68 flow paths. The first flow path 64 through the top port 82 is unaffected by the rotation of handle 56 and always remains in communication with the central chamber 50. The second flow path 66 is in communication with the entry port 44 of the body portion 40 being in communication with the housing 38 of the catheter hub assembly 14 and the third flow path 67 is in communication with the exit port 46. The fourth flow path 68 is in communication with the injection port 48. Preferably, arrows showing the direction of the flow path are molded on an upper surface 76 of the handle 56 (see Fig. 7A). The arrows correspond to each of the second 66, third 67 and fourth 68 flow paths and show whether fluid will flow.

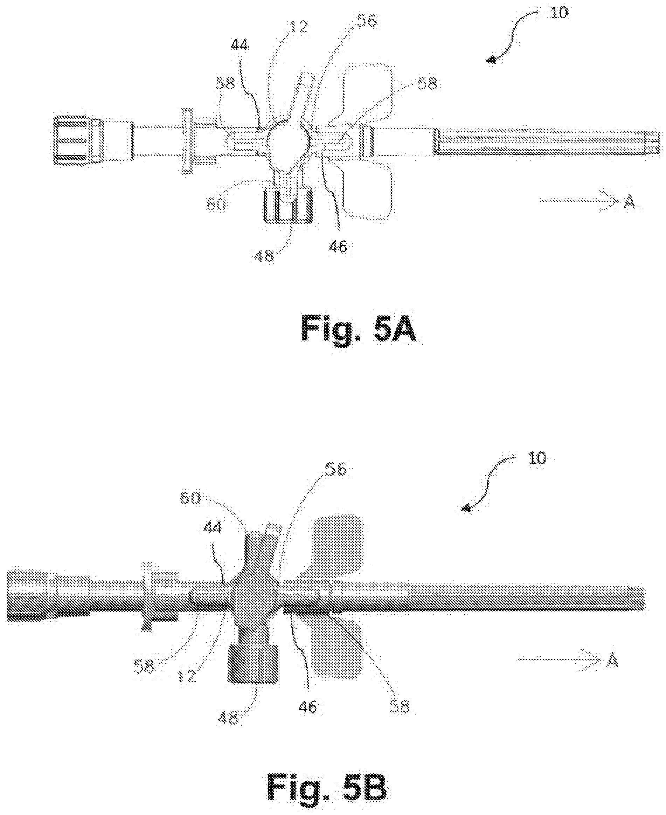

The status of passage of fluid through the first 64, second 66, third 67 and fourth 68 flow paths by use of the rotational handle 56 is discussed with reference to Figs. 5A to 5D. It is to be understood that in the illustrated Figs. 5A to 5D the intravenous catheter apparatus 10 is without the needle 18, thus allowing free rotation of the handle 56 and the three-way stopcock assembly 12 relative to the catheter hub assembly 14.

In a rotational position as shown in Fig. 5A, when the second portion 60 of the handle 56 points toward the injection port 48, fluid can simultaneously flow between all of the first 64, second 66, third 67 and fourth 68 flow paths at one time. In this position, the passage of fluids from each of the top port 82, entry port 44, and injection port 48 is open and allow the fluids to make an exit through the exit port 46.

Referring now to Fig. 5B, when the second portion 60 of the handle 56 points toward the opposite of injection port 48, fluid can simultaneously flow between only the first 64, second 66 and third 67 flow paths at one time. In this position, passage of fluid through the injection port 48 is blocked.

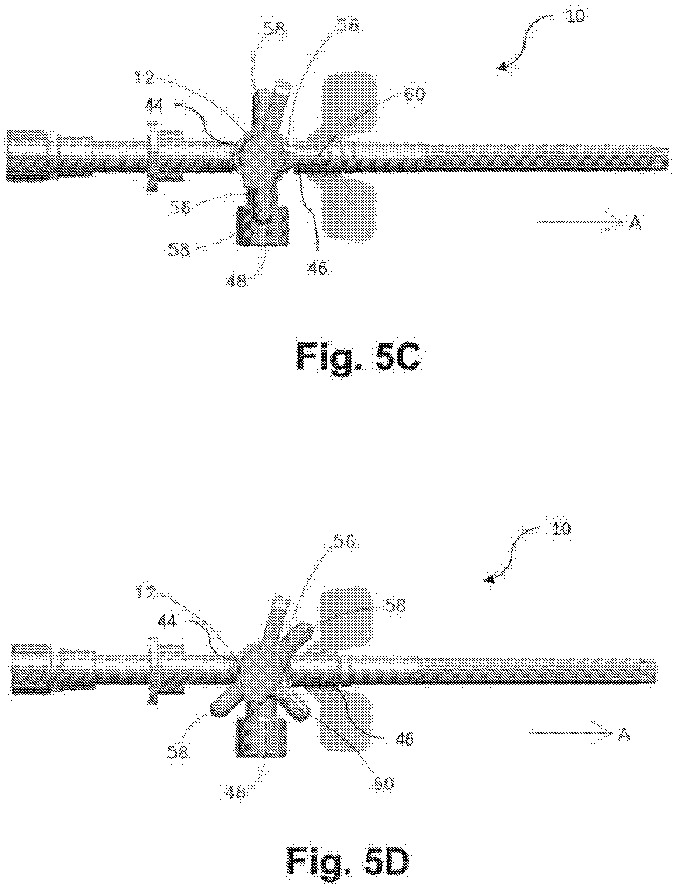

Referring now to Fig. 5C, when the second portion 60 of the handle 56 points toward the exit port 46, fluid can simultaneously flow between only the first 64, third 67 and fourth 68 flow paths at one time. In this position, passage of fluid through the entry port 44 is blocked.

Referring now to Fig. 5D, when the second portion 60 of the handle 56 is not pointing towards any one of ports 44, 46, or 48, fluid is blocked to pass through all of the second 66, third 67 and fourth 68 flow paths. In this position, the handle 56 is configured to be rotatable at each 45° intervals, wherein passage of fluid through the entry port 44, exit port 46 and injection port 48 is blocked.

The integrated three-way stopcock assembly 12, thus, has at least four fluid flow ports capable of providing four paths for fluid to flow simultaneously at one time, including the arrangement of providing at least two fluid flow paths to flow simultaneously.

The intravenous catheter apparatus 10 is provided with a click-feature giving a detent feeling when the three-way stopcock assembly 12 is rotated inside the catheter hub assembly 14. While rotating the three-way stopcock assembly 12 via the handle 56, a user can experience the click with respect to locking and unlocking of the flow paths 66, 67, and 68 by rotational displacement of the first 70, second 72 and third 74 openings of the three-way stopcock assembly 12 with regard to the respective ports 44, 46, and 48 of the catheter hub assembly 14. It is to be understood that the intravenous catheter apparatus 10 can also be provided without the click feature.

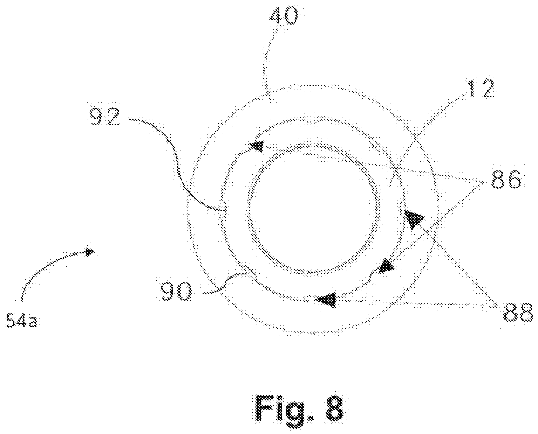

For the click-feature as shown in Figs. 7A and 8, in one embodiment, an inner wall 90 of the bottom portion 54 of the catheter hub assembly 14 is provided with projections 86. Grooves 88 matching with said projections 86 are provided in an outer wall 92 of the three-way stopcock assembly 12 in the bottom portion 54a, preferably, not being limited to, at an alternating interval of 45°. Such intervals or steps of rotation are at half the angle between the injection port 48 and the entry port 44, if these two ports 44 and 48 are at a right angle as shown in the Fig. 6B. However, the steps may be even smaller, as long as they are at angles equal to half the angle between the injection port 48 and the entry port 44. It is pointed out that in any embodiment incorporating a click-feature, a minimal solution is to provide only one protrusion or likewise element and only so much grooves as rotationally selectable ports are present, which here are three, i.e. the entry port 44, the exit port 46, and the injection port 48. This way the production costs for creating the grooves are reduced. Also, rotating the handle such that no groove is engaged will result in the blocking of all of the rotationally selectable ports 44, 46, and 48. As an alternative, there may be one additional groove for the latter purpose, which additional groove ensures that the user receives a feedback that all ports 44, 46, and 48 are indeed and securely blocked.

While rotating the handle 56 at each 45° interval as illustrated in Fig. 5D, the grooves 88 mate with the projections 86 resulting in a detent feeling ensuring proper alignment of the handle 56 with respect to the fluid paths 66, 67, and 68 to ascertain locked or unlocked status of the paths by displacing the openings 70, 72, and 74 with regard to the respective ports 44, 46, and 48 such that they are blocked or unblocked by the cylindrical wall at the bottom portion 54 of the catheter hub assembly 14. In this embodiment, the combination of projections 86 and grooves 88, not being limited to, is arranged in the bottom portions 54a and 54 of the three-way stopcock assembly 12 and the body portion 40 of the catheter hub assembly 14, respectively. However, this combination can be arranged anywhere in between the three-way stopcock assembly 12 and the catheter hub assembly 14.

In an alternative embodiment, the inner wall 90 of the bottom portion 54 of the catheter hub assembly 14 is provided with grooves 88. Projections 86 matching with said grooves 88 are provided in the outer wall 92 of the three-way stopcock assembly 12 in a bottom portion 54a thereof. This combination also can be arranged anywhere in between the three-way stopcock assembly 12 and the catheter hub assembly 14.

In a further embodiment, the combination of projections 86 and grooves 88 can also be arranged between a bottom face 94 of the bottom portion 54a of the three-way stopcock assembly 12 (see Fig. 7B) and a top face 96 of the bottom portion 54 of the body portion 40 (see Fig. 6C), respectively. The use of projections 86 and grooves 88 can also be interchanged and/or a combination thereof can be used to attain the click-feature. Also, providing only one projection 86 is sufficient in all previously described variations to achieve the detent feeling and the necessary positional feedback, as long as there are several grooves 88 corresponding to the distinct locked and unlocked rotational positions of the three-way stop cock assembly 12. Referring now to Fig. 9, a further embodiment to attain the click-feature of the handle 56 is illustrated. In this arrangement, a bottom face 94a of the bottom portion 54b of the handle 56 is provided with a mating member 98. The mating member 98, not being limited to, is a protrusion formed by a combination of a spring and ball which is arranged within a recess 100 provided in the bottom face 94a of the handle 56. Grooves 88 matching with said mating member 98 are arranged in a top face 96a of the top portion 52 of the body portion 40 of the catheter hub assembly 14. While rotating the three-way stopcock assembly 12 by the handle 56 the mating member 98 having the spring loaded ball remains pre-compressed until it comes into contact with a groove 88 provided in the body portion 40 into which it is then pushed by its spring. Hence, the spring urges the ball into one of the grooves 88 upon rotational alignment. While rotating the handle 56, the mating of the mating member 98 and the grooves 88 generates a click-feature ensuring proper alignment of the three-way stopcock assembly 12 with respect to the fluid paths 66, 67, and 68 to ascertain locked or unlocked status of the fluid paths 66, 67, and 68.

As becomes apparent from Figs. 4B and 9, the flow regulating member 80 is a cylindrical elastic element surrounding the blind hole 62 and covering the side opening 63 from the central chamber 50 side of the blind hole 62. This way fluid can be forced from the outside through the top port 82 into the blind hole 62 and through the side opening 63 while acting against the resilient force of the flow regulating member 80 to reach the central chamber 50. On the other hand, fluid being forced from the central chamber 50 towards the side opening 63 will only apply additional sealing force to the flow regulating member 80 covering the side opening 63 such that a back flow is prevented reliably. In order to ensure that the flow regulating member 80 remains in place in order to reliably execute the above explained function, the two protrusions 51 extend from the bottom portion 54 of the body portion 40 of the catheter hub assembly 14 within the bottom portion 54a of the three-way stopcock assembly 12 and towards the flow regulating member 80 in order to stop through abutment the flow regulating member 80 from slipping off.

Further, the above explained click-feature may be integrated into the snap-fit, in particular into the protrusion ring 57 and the corresponding ring groove 41 for production cost saving reasons.

Although this invention has been disclosed in the context of certain preferred embodiments and examples, it will be understood by those skilled in the art that the present invention extends beyond the specifically disclosed embodiments to other alternative embodiments and/or uses of the invention and obvious modifications and equivalents thereof. Thus, from the foregoing description, it will be apparent to one of ordinary skill in the art that many changes and modifications can be made thereto without departing from the scope of the invention.

Accordingly, it is not intended that the scope of the foregoing description be limited to the exact description set forth above, but rather that such description be construed as encompassing such features that reside in the present invention, including all the features and embodiments that would be treated as equivalents thereof by those skilled in the relevant art.

Thus, it is intended that the scope of the present invention herein disclosed should not be limited by the particular disclosed embodiments described above but should be determined only by a fair reading of the appended claims.

### LIST OF REFERENCE NUMERALS

- 10: Intravenous catheter apparatus
- 12: three-way stopcock assembly
- 14: catheter hub assembly
- 16: catheter tube
- 18: needle
- 20: needle shaft
- 22: needle tip
- 24: distal section
- 26: proximal section
- 28: needle hub
- 30: proximal end of the needle shaft
- 30a: proximal end of the catheter hub assembly
- 32: distal end of the needle shaft
- 32a: distal end of the catheter hub assembly
- 32b: distal end of the wing housing
- 34: enlargement
- 36: wing housing
- 38: housing
- 40: body portion
- 41: ring groove
- 42: needle guard
- 44: entry port
- 46: exit port
- 48: injection port
- 50: central chamber
- 51: protrusion(s)
- 52: top portion of the catheter hub assembly
- 52a: top portion of the three-way stopcock assembly
- 52b: top portion of the handle
- 54: bottom portion of the catheter hub assembly
- 54a: bottom portion of the three-way stopcock assembly
- 54b: bottom portion of the handle
- 56: handle
- 57: protrusion ring
- 58: axial portion
- 59: blind hole protrusion
- 60: horizontal portion
- 62: bore
- 63: side opening
- 64: first flow path
- 66: second flow path
- 67: third flow path
- 68: fourth flow path
- 70: first opening
- 72: second opening
- 74: third opening
- 76: upper surface
- 78: cap
- 80: flow regulating member
- 82: top port
- 84: opening
- 86: projection(s)
- 88: grooves
- 90: inner wall
- 92: outer wall
- 94: bottom face of the bottom portion of the three-way stopcock assembly
- 94a: bottom face of the handle
- 96: top face of the bottom portion of the body portion of the catheter hub assembly
- 96a: top face of the top portion of the body portion of the catheter hub assembly
- 98: mating member
- 100: recess

## Claims

1. An intravenous catheter apparatus (10) comprising:
an integrated three-way stopcock assembly (12), and
a catheter hub assembly (14) having a proximal end (30a) configured to be connected to a needle hub (28) having a needle (18), and having a distal end (32a) configured to be connected to a catheter tube (16), wherein a straight needle (18) is capable of passing through said proximal end (30a) and distal end (32a) of the catheter hub assembly (14), in a ready position of the intravenous catheter apparatus (10), said catheter hub assembly (14) includes:
a wing housing (36) at the distal end (32a), a housing (38) at the proximal end
(30a), and a body portion (40) in between housing said three-way stopcock assembly (12);
wherein said body portion (40) comprises an entry port (44) in communication with said housing (38), an exit port (46) in communication with said wing housing (36), wherein the entry port (44) and the exit port (46) extend along a horizontal line in an axial direction (A), and an injection port (48), wherein all of these ports (44, 46, 48) are confluent at a central chamber (50) configured within the body portion (40);
wherein said three-way stopcock assembly (12) has a top portion (52a) and an opposing bottom portion (54a), both along a vertical line perpendicular to the horizontal line, as well as a handle (56) configured in said top portion (52a), wherein the handle (56) has a first portion (58) parallel to the horizontal line and a second portion (60) parallel to the injection port (48), in the ready position, and
wherein the three-way stopcock assembly (12) has rotation capabilities relative to the body portion (40) of the catheter hub assembly (14),
**characterized in that**
a bore (62) is provided in the handle (56), wherein the bore (62) has an opening (84) in a top portion (52) thereof communicating with said central chamber (50) and defining a top port (82),
wherein a flow regulating member (80) is arranged in between the opening (86) of the top port (84) and the central chamber (50) such that it allows fluid passage only from the outside to the central chamber (50) and prevents back flow of fluid from the central chamber (50) to the outside, and
wherein the bore (62) is a blind hole (62) with a side opening (63) at the bottom which communicates with the central chamber (50) and the flow regulating member (80) is an elastic element covering the side opening (63) from the central chamber side of the blind hole (62).

2. The intravenous catheter apparatus (10) as claimed in claim 1, wherein the body portion (40) is molded as one piece.

3. The intravenous catheter apparatus (10) as in any one of the preceding claims, wherein a direction of extension of the first portion (58) of the handle (56) corresponds to a direction of extension of a first opening (70) and a second opening (72) in the bottom portion (54a) of the three-way stopcock assembly (12), and a direction of extension of the second portion (60) of the handle (56) corresponds to a direction of extension of a third opening (74) in the bottom portion (54a) of the three-way stopcock assembly (12).

4. The intravenous catheter apparatus (10) as claimed in claim 1, wherein the catheter hub assembly (14) has at least one protrusion (51) in a bottom portion (54) extending within the bottom portion (54a) of the three-way stopcock assembly (12) and towards the flow regulating member (80) so as to prevent an accidental displacement of the flow regulating member (80) and uncovering of the side opening (63).

5. The intravenous catheter apparatus (10) as claimed in any one of the preceding claims, wherein a click-feature (86, 88; 98, 88) is provided between the three-way stopcock assembly (12) and the catheter hub assembly (14).

6. The intravenous catheter apparatus (10) as claimed in claim 5, wherein the click-feature comprises at least one protrusion (86; 98) and several grooves (88) on one of the three-way stopcock assembly (12) and the catheter hub assembly (14).

7. The intravenous catheter apparatus (10) as claimed in claim 6, wherein the protrusion is a mating member (98) comprising a spring and a ball, wherein the spring is configured to urge the ball into the grooves (88) upon a corresponding rotational alignment of the three-way stopcock assembly (12) with regard to the catheter hub assembly (14).

## Patentansprüche

1. Intravenöse Kathetervorrichtung (10) umfassend:
eine integrierte Dreiwegehahn-Anordnung (12) und/oder
eine Katheterhub-Anordnung (14), die ein proximales Ende (30a) aufweist, das so konfiguriert ist, dass es mit einem Nadelhub (28) verbunden wird, der eine Nadel (18) aufweist, und die ein distales Ende (32a) aufweist, das so konfiguriert ist, dass es mit einem Katheterschlauch (16) verbunden ist, wobei in einer betriebsbereiten Position der intravenösen Kathetervorrichtung (10) eine gerade Nadel (18) durch das proximale Ende (30a) und das distale Ende (32a) der Katheterhub-Anordnung (14) treten kann, die Katheterhub-Anordnung (14) umfassend:
ein Flügelgehäuse (36) am distalen Ende (32a), ein Gehäuse (38) am proximalen Ende
(30a) und einen Körperabschnitt (40) dazwischen, der die Dreiwegehahn-Anordnung (12) aufnimmt;
wobei der Körperabschnitt (40) einen Eingangsanschluss (44), der mit dem Gehäuse (38) in Verbindung steht, einen Ausgangsanschluss (46), der mit dem Flügelgehäuse (36) in Verbindung steht, wobei sich der Eingangsanschluss (44) und der Ausgangsanschluss (46) entlang einer horizontalen Linie in axialer Richtung (A) erstrecken, und einen Injektionsanschluss (48) umfasst, wobei alle drei Anschlüsse (44, 46, 48) an eine zentralen Kammer (50) zusammenfließen, die in dem Körperabschnitt (40) konfiguriert ist;
wobei die Dreiwegehahn-Anordnung (12) einen oberen Abschnitt (52a) und einen gegenüberliegenden unteren Abschnitt (54a), beide entlang einer vertikalen Linie, die senkrecht zur horizontalen Linie verläuft, sowie einen Griff (56) aufweist, der in dem oberen Abschnitt (52a) konfiguriert ist, wobei der Griff (56) in der betriebsbereiten Position einen ersten Abschnitt (58) parallel zur horizontalen Linie und einen zweiten Abschnitt (60) parallel zum Injektionsanschluss (48) aufweist, und wobei die Dreiwegehahn-Anordnung (12) bezüglich des Körperabschnitts (40) der Katheterhub-Anordnung (14) drehfähig ist,
**dadurch gekennzeichnet, dass**
eine Bohrung (62) im Griff (56) vorgesehen ist, wobei die Bohrung (62) eine Öffnung (84) in einem oberen Abschnitt (52) davon aufweist, der mit der zentralen Kammer (50) in Verbindung steht und einen oberen Anschluss (82) umgrenzt,
wobei eine strömungsregulierende Einheit (80) zwischen der Öffnung (86) des oberen Anschlusses (84) und der zentralen Kammer (50) angeordnet ist, sodass es einen Flüssigkeitsdurchfluss nur von außen in die Zentralkammer (50) ermöglicht und einen Rückfluss von Flüssigkeit von der Zentralkammer (50) nach außen verhindert, und
wobei die Bohrung (62) ein Sackloch (62) mit einer Seitenöffnung (63) am unteren Ende ist, die mit der zentralen Kammer (50) in Verbindung steht, und die strömungsregulierende Einheit (80) ein elastisches Element ist, das die Seitenöffnung (63) von der Seite der zentralen Kammer des Sacklochs (62) bedeckt.

2. Intravenöse Kathetervorrichtung (10) nach Anspruch 1, wobei der Körperabschnitt (40) in einem Stück gegossen ist.

3. Intravenöse Kathetervorrichtung (10) nach einem der vorhergehenden Ansprüche, wobei eine Richtung, in der sich der erste Abschnitt (58) des Griffs (56) erstreckt, einer Richtung entspricht, in der sich eine erste Öffnung (70) und eine zweite Öffnung (72) im unteren Abschnitt (54a) der Dreiwegehahn-Anordnung (12) erstreckt, und eine Richtung, in der sich der zweite Abschnitt (60) des Griffs (56) erstreckt, einer Richtung entspricht, in der sich eine dritte Öffnung (74) im unteren Abschnitt (54a) der Dreiwegehahn-Anordnung (12) erstreckt.

4. Intravenöse Kathetervorrichtung (10) nach Anspruch 1, wobei die Katheterhub-Anordnung (14) mindestens einen Vorsprung (51) in einem unteren Abschnitt (54) aufweist, der sich innerhalb des unteren Abschnitts (54a) der Dreiwegehahn-Anordnung (12) und in Richtung der strömungsregulierenden Einheit (80) erstreckt, um ein versehentliches Verschiebendes strömungsregulierenden Elements (80) und Aufdecken des Seitenöffnung (63) zu verhindern.

5. Intravenöse Kathetervorrichtung (10) nach einem der vorhergehenden Ansprüche, wobei ein Klickmerkmal (86, 88; 98, 88) zwischen der Dreiwegehahn-Anordnung (12) und der Katheterhub-Anordnung (14) vorgesehen ist.

6. Intravenöse Kathetervorrichtung (10) nach Anspruch 5, wobei das Klickmerkmal mindestens einen Vorsprung (86; 98) und mehrere Rillen (88) auf einer der Dreiwegehahn-Anordnungen (12) und der Katheterhub-Anordnung (14) umfasst.

7. Intravenöse Kathetervorrichtung (10) nach Anspruch 6, wobei der Vorsprung eine passende Einheit (98) ist, die eine Feder und einen Ball umfasst, wobei die Feder so konfiguriert ist, dass der Ball bei einer entsprechenden Drehausrichtung der Dreiwegehahn-Anordnung (12) bezüglich der Katheterhub-Anordnung (14) in die Rillen (88) gedrängt wird.

## Revendications

1. Appareil de cathéter intraveineux (10) comprenant :
un ensemble de robinet d'arrêt trois voies intégré (12), et
un ensemble (14) de raccord de cathéter ayant une extrémité proximale (30a) configurée pour être connectée à un raccord (28) d'aiguille ayant une aiguille (18), et ayant une extrémité distale (32a) configurée pour être connectée à un tube (16) de cathéter, dans lequel une aiguille droite (18) est apte à passer à travers lesdites extrémité proximale (30a) et extrémité distale (32a) de l'ensemble (14) de raccord de cathéter, dans une position prête de l'appareil de cathéter intraveineux (10), ledit ensemble (14) de raccord de cathéter inclut :
un logement (36) d'ailette à l'extrémité distale (32a), un logement (38) à l'extrémité proximale (30a), et une partie (40) de corps entre ceux-ci logeant ledit ensemble de robinet d'arrêt trois voies (12) ;
dans lequel ladite partie (40) de corps comprend un orifice d'entrée (44) en communication avec ledit logement (38), un orifice de sortie (46) en communication avec ledit logement (36) d'ailette, dans lequel l'orifice d'entrée (44) et l'orifice de sortie (46) s'étendent le long d'une ligne horizontale dans un sens axial (A), et un orifice d'injection (48), dans lequel tous ces orifices (44, 46, 48) sont confluents au niveau d'une chambre centrale (50) configurée à l'intérieur de la partie (40) de corps ;
dans lequel ledit ensemble de robinet d'arrêt trois voies (12) a une partie supérieure (52a) et une partie inférieure (54a) opposée, les deux le long d'une ligne verticale perpendiculaire à la ligne horizontale, ainsi qu'un poignée (56) configurée dans ladite partie supérieure (52a), dans lequel la poignée (56) a une première partie (58) parallèle à la ligne horizontale et une deuxième partie (60) parallèle à l'orifice d'injection (48), dans la position prête, et
dans lequel l'ensemble de robinet d'arrêt trois voies (12) a des capacités de rotation par rapport à la partie (40) de corps de l'ensemble (14) de raccord de cathéter,
**caractérisé en ce que**
un alésage (62) est prévu dans la poignée (56), dans lequel l'alésage (62) a une ouverture (84) dans une partie supérieure (52) de celui-ci communiquant avec ladite chambre centrale (50) et définissant un orifice supérieur (82),
dans lequel un élément (80) de régulation de flux est agencé entre l'ouverture (86) de l'orifice supérieur (84) et la chambre centrale (50) de telle sorte qu'il permet un passage de fluide uniquement de l'extérieur jusqu'à la chambre centrale (50) et empêche un reflux de fluide de la chambre centrale (50) jusqu'à l'extérieur, et
dans lequel l'alésage (62) est un trou borgne (62) avec une ouverture latérale (63) au niveau du fond qui communique avec la chambre centrale (50) et l'élément (80) de régulation de flux est un élément élastique recouvrant l'ouverture latérale (63) depuis le côté de chambre centrale du trou borgne (62).

2. Appareil de cathéter intraveineux (10) selon la revendication 1, dans lequel la partie (40) de corps est moulée comme une seule pièce.

3. Appareil de cathéter intraveineux (10) selon l'une quelconque des revendications précédentes, dans lequel un sens d'extension de la première partie (58) de la poignée (56) correspond à un sens d'extension d'une première ouverture (70) et d'une deuxième ouverture (72) dans la partie inférieure (54a) de l'ensemble de robinet d'arrêt trois voies (12), et un sens d'extension de la deuxième partie (60) de la poignée (56) correspond à un sens d'extension d'une troisième ouverture (74) dans la partie inférieure (54a) de l'ensemble de robinet d'arrêt trois voies (12).

4. Appareil de cathéter intraveineux (10) selon la revendication 1, dans lequel l'ensemble (14) de raccord de cathéter a au moins une saillie (51) dans une partie inférieure (54) s'étendant à l'intérieur de la partie inférieure (54a) de l'ensemble de robinet d'arrêt trois voies (12) et vers l'élément (80) de régulation de flux de manière à empêcher un déplacement accidentel de l'élément (80) de régulation de flux et un découvrement de l'ouverture latérale (63).

5. Appareil de cathéter intraveineux (10) selon l'une quelconque des revendications précédentes, dans lequel une caractéristique d'encliquetage (86, 88 ; 96, 88) est prévue entre l'ensemble de robinet d'arrêt trois voies (12) et l'ensemble (14) de raccord de cathéter.

6. Appareil de cathéter intraveineux (10) selon la revendication 5, dans lequel la caractéristique d'encliquetage comprend au moins une saillie (86 ; 98) et plusieurs rainures (88) sur un de l'ensemble de robinet d'arrêt trois voies (12) et de l'ensemble (14) de raccord de cathéter.

7. Appareil de cathéter intraveineux (10) selon la revendication 6, dans lequel la saillie est un élément d'appariement (98) comprenant un ressort et une bille, dans lequel le ressort est configuré pour pousser la bille dans les rainures (88) lors d'une alignement en rotation correspondant de l'ensemble de robinet d'arrêt trois voies (12) par rapport à l'ensemble (14) de raccord de cathéter.
